# EUROPÄISCHE PATENTSCHRIFT

(11) **EP 2 249 715 B1**
(45) Veröffentlichungstag und Bekanntmachung des Hinweises auf die Patenterteilung: **08.01.2014**
(21) Anmeldenummer: 09707373.8
(22) Anmeldetag: 06.02.2009
(51) Int. Cl.: A61B 17/16

(54) **ZERLEGBARES CHIRURGISCHES INSTRUMENT**
SURGICAL INSTRUMENT WHICH CAN BE DISASSEMBLED
INSTRUMENT CHIRURGICAL DÉMONTABLE

(30) Priorität: 06.02.2008 DE 202008001675 U
(43) Veröffentlichungstag der Anmeldung: 17.11.2010
(73) Patentinhaber: Waldemar Link GmbH & Co. KG, 22339 Hamburg (DE)
(72) Erfinder: KÜHN, Matthias, 79215 Elzach (DE)
(74) Vertreter: Heisel, Wolfgang
(86) Internationale Anmeldenummer: PCT/IB2009/000224
(87) Internationale Veröffentlichungsnummer: WO 2009/098589

(56) Entgegenhaltungen:
- EP-A- 1 212 983
- DE-A1- 19 748 369
- DE-A1- 19 915 427
- DE-U1- 20 103 630

## Beschreibung

Die Erfindung bezieht sich auf ein chirurgisches Instrument, bestehend aus einem Grundelement, das aus einem ersten Griffteil sowie einen mit dem Griffteil verbundenen Führungselement besteht, wobei sich das Führungselement von dem Griffelement aus axial erstreckt sowie einem weiteren Griffteil, das an dem Grundelement angeordnet ist. Zudem ist ein auf dem Führungselement in dessen axialer Erstreckung hin- und hergleitendes Schiebeelement mit einem proximalen und einem distalen Ende vorgesehen, wobei das Schiebeelement an seinem proximalen Ende mit dem weiteren Griffteil über ein Kopplungselement verbunden ist. Zudem weist das Kopplungselement ein Mitnehmerteil auf, das ein mit dem Mitnehmerteil korrespondierendes Verbindungsglied, das an dem Schiebeelement angeordnet ist, entsprechende Schwenkbewegungen des weiteren Griffteils hin- und herbewegt, sowie ein Funktionselement, das mit dem Schiebeelement am distalen Ende gekoppelt ist. Das Funktionselement ist unverlierbar mit dem Schiebeelement gekoppelt und an dem Führungselement drehbeweglich angeordnet.

### Definition

Gattungsmässige Instrumente werden auch in die Gruppe der Schiebeelementinstrumente eingeordnet. Solche Schiebeelementinstrumente finden insbesondere im Bereich der minimal-invasiven endoskopischen Eingriffen Anwendung. In der Regel weisen sie einen dünnen, lang gestreckten Schaft auf, der einen Durchmesser zwischen 5mm und 15mm aufweist. An seinem distalen Ende ist als Funktionselement ein Arbeitswerkzeug vorgesehen. Dieses Arbeitswerkzeug weist in der Regel ein bewegbares Werkzeugelement auf, das insbesondere als Zange oder Schneideinrichtung ausgebildet ist. Die Bewegung dieses Werkzeugelements wird durch die am proximalen Ende des Schaftes vorgesehene Betätigungseinrichtung bewirkt. In der Regel handelt es sich dabei um eine Scherengriffeinrichtung mit einer seitlich angeordneten feststehenden Branche und daran verschwenkbar gelagerten Griffbranchen beziehungsweise Griffteilen.

Zwischen dem Arbeitswerkzeug und der Griffbranche ist ein Kraftübertragungselement angeordnet. Es dient dazu, die durch die Griffbranche aufgebrachte Kraft auf das Werkzeugelement auszuüben.

In der nachfolgenden Beschreibung werden für das chirurgische Instrument zur Positionsangabe die Begriffe "proximal" und "distal" verwendet. Der Begriff "proximal" bezieht sich auf den Bereich des Instruments, der sich weg von dem Patienten befindet, wohingegen der Begriff "distal" sich auf den Bereich bezieht, der dem Patienten zugeordnet ist. Dies bedeutet, dass das Instrument von einem Anwender im proximalen Bereich gegriffen und bedient wird.

### Stand der Technik

Aus dem Stand der Technik sind eine Vielzahl von chirurgischen Instrumenten, die als Schaft- oder Schiebeelementinstrument ausgebildet sind, bekannt.

So ist beispielsweise aus der DE 29815846 U (OLYMPUS WINTER ) 03.09.1998 ein chirurgisches Schiebeelementinstrument bekannt, das aus einer feststehenden Griffbranche besteht, an dessen distalen Ende ein Werkzeugelement angeordnet ist. Das Werkzeugelement ist in Form einer Schere oder Zange ausgebildet und wird über ein Kugelgelenk, angeordnet am proximalen Ende des Schiebeelementinstruments, bedient. Ein Seilzug, der in dem Schaft angeordnet ist, dient zur Kraftübertragung.

Ferner ist aus der DE 4341734 C (AESCULAP AG) 08.12.1993 ein Instrument für chirurgische Zwecke bekannt. Auch dieses besteht aus einer feststehenden Griffbranche und eine zu diesem verschwenkbar gelagerten weiteren Griffbranche, wobei die weitere Griffbranche über ein Kraftübertragungsmittel die aufgebrachte Kraft auf das am distalen Ende angeordnete Werkzeugelement überträgt. Das Werkzeugelement selbst ist als Schere oder Zange ausgebildet. Zusätzlich dient das hier beschriebene chirurgische Instrument dazu, auf das distale Ende des Werkzeugelements eine entsprechende elektrische Spannung aufzubringen.

Aus der DE 9307621 U (AESCULAP AG) 19.05.1993 ist ein chirurgisches Stanzinstrument bekannt. Dieses chirurgische Stanzinstrument weist wiederum eine feststehende Griffbranche auf, an der verschwenkbar gelagert eine bewegliche weitere Griffbranche angeordnet ist. Die bewegliche Griffbranche ist wiederum über ein Kopplungselement mit einem Schiebeelement verbunden, wobei das Schiebeelement auf einem Führungselement, das sich axial in Längsrichtung des chirurgischen Instruments erstreckt, hin- und herbewegt. Als Funktionselement, das am distalen Ende des Schiebeelements angeordnet ist, ist das Werkzeugelement in Form einer Stanze angeordnet. Die bewegliche Griffbranche weist ein Mitnehmerteil auf, das mit einem Verbindungsteil, das in dem Schiebeelement angeordnet ist, zusammenwirkt. Aus Reinigungsgründen ist das Schiebeelement von dem Führungselement vollständig entfernbar. Die verschwenkbar gelagerte Griffbranche ist unverlierbar an dem Grundelement, das auch die weitere Griffbranche umfasst, angeordnet.

So ist auch aus der DE 29922271 (WEINMANN GMBH) 17.12.1999 ein chirurgisches Instrument mit ebenfalls einem Scherengriff bekannt. Der Scherengriff wird gebildet aus einer feststehenden Griffbranche, die als Teil des Grundelements ausgebildet ist, auf dem auch ein Schiebeelement gleitet, sowie einer weiteren beweglichen Griffbranche, die sich relativ zu der ersten Griffbranche bewegt. An dem freien distalen Ende ist ein Werkzeugelement in Form einer Knochenstanze angeordnet.

Aus der US 6126674 A (PETER JANZEN) 28.10.1998 ist ebenfalls ein chirurgisches Instrument in der Ausbildung eines Schiebeelementinstruments ausgebildet. Die erste Griffbranche ist fest mit dem Grundelement verbunden, auf dessen ein Schiebeelement gleitet. Der Schiebeelement ist wiederum mit einem Funktionselement beziehungsweise über ein Konnektierungselement mit einer weiteren an dem Grundelement beweglichen Griffbranche verbunden.

Aus der US 5851214 A (SCOTT W. LARSEN, CHRISTOPHER MCDONNEL, SCOTT W. REED) 16.09.1996 ist ein chirurgisches Instrument, das insbesondere für endoskopische Eingriffe geeignet ist, dargestellt. Dieses chirurgische Instrument, in der Ausbildung eines Chonchotoms, weist ein Grundelement auf, an dem ein erstes Griffteil fest verbunden ist. Das Grundelement umfasst weiter ein Führungselement, das sich axial von dem ersten Griffteil weg erstreckt. Zudem ist ein weiteres Griffteil vorgesehen, das beweglich an dem Grundelement angeordnet ist. Auf dem Führungselement selbst ist in axialer Erstreckung hin- und hergleitend ein Schiebeelement angeordnet, das jeweils ein proximales und distales Ende aufweist. An dem proximalen Ende ist das Schiebeelement über ein Kopplungselement mit dem weiteren Griffteil verbunden. Das Kopplungselement selbst umfasst ein Mitnehmerteil, wobei an dem Schiebeelement ein Verbindungsteil angeordnet ist, das die Eigenschaft hat, die von dem weiteren Griffteil ausgeübte Schwenkbewegung beziehungsweise Kraft auf das Schiebeelement zu übertragen.

Aus der DE 10 2004 009 200 A1 (Larl Storz GmbH & Co. KG) 25.02.2004 ebenfalls ein chirurgisches Instrument für den endoskopischen Einsatz bekannt. An dem distalen Ende des Instruments ist ein Funktionselement in der Ausbildung eines Schneid- oder Klemmmechanismus bekannt, der über ein Schiebeelement, das wiederum mit einer Griffbranche gekoppelt ist, zusammenwirkt. Das Schiebeelement ist unverlierbar an das Funktionselement gekoppelt, wobei der proximale Teil von der Griffbranche entkoppelt werden kann, um eine Reinigung zu gewährleisten.

Aus der US 5 507 772 (Shutt et al) 20.05.1994 ist ein chirurgisches Schiebeelementinstrument bekannt, das an seinem distalen Ende ein Funktionselement, beispielsweise in der Ausbildung einer Schere aufweist. Dieses Funktionselement ist wiederum gekoppelt mit einem Schiebeelement, das mit einer scherenartigen Griffbranche zusammenwirkt. Durch Bewegen des scherenartigen Griffbranchenmechanismus kann das Funktionselement geöffnet und geschlossen werden. Da das Instrument insbesondere für den endoskopischen Einsatz vorgesehen ist, ist eine Reinigung notwendig. Um die Reinigung des Bereichs des Schiebeelements vorzusehen, ist dieser derart entfernbar, dass das distale Teil weiterhin drehgelenkig mit dem Funktionselement verbunden bleibt, wohingegen das proximale Ende durch Betätigen eines Entriegelungsmechanismus von der Griffbranche entkoppelbar ist. Eine passgenaue Zuordnung insbesondere bei Reinigungsvorgängen von mehreren solchen chirurgischen Schiebeelementinstrumenten ist somit gewährleistet.

Auch die DE 199 15 427 A1 (Karl Storz GmbH & Co. KG) 6.04.1999 zeigt ein medizinisches Instrument in der Ausbildung eines Schiebeelementinstruments. Es unterscheidet sich im wesentlichen von dem in der US 5 507 772 gezeigten Ausbildung dadurch, dass der Entkoppelungsmechanismus des Schiebeelements von der Griffbranche anders gelöst ist. Die Kopplung des Schiebeelements erfolgt über ein Mitnahmeelement der ersten Griffbranche, das beweglich am Grundkörper des Instruments angeordnet ist. In einer bestimmten Stellung ist das Schiebeelement aus diesem Mitnahmeelement herausnehmbar. Um zu verhindern, dass ein Lösen während der Nutzung des Instruments geschieht, ist ein Sicherungselement vorgesehen, das die Verschieblichkeit des Schiebeelements beschränkt. Das Sicherungselement ist im Querschnitt U-förmig ausgebildet und kann über den Entkopplungsbereich zur Sicherung des Schiebeelements geklappt werden. Zusätzliche Raststifte sichern das ungewollte Öffnen des Sicherungselements.

Aus der EP 1 212 983 A2 (Tontorra Medizintechnik GmbH) ist ein chirurgisches Instrument bekannt, das die Merkmale des Oberbegriffs von Anspruch 1 aufweist und das aus einem Grundelement, das aus einem ersten Griffteil sowie aus einem mit dem Griffteil verbundenen Führungselement besteht, wobei das Führungselement sich von dem Griffelement aus axial erstreckt sowie einem weiteren Griffteil, das an dem Grundelement schwenkbar angeordnet ist. Ferner ist ein Schiebeelement mit einem proximalen und einem distalen Ende vorgesehen, das auf dem Führungselement in dessen axialer Erstreckung hin- und hergleitbar angeordnet ist, wobei das Schiebeelement an seinem proximalen Ende mit einem weiteren Griffteil über ein Kopplungselement verbunden ist, das Kopplungselement ein Mitnehmerteil umfasst, das ein mit dem Mitnehmerteil korrespondierendes Verbindungsteil, das an dem Schiebeelement angeordnet ist, entsprechend der Schwenkbewegung des weiteren Griffteils hin- und herbewegt. Das Mitnehmerteil ist zusätzlich über eine Drehachse mit dem weiteren Griffteil gekoppelt. Zur Begrenzung der Bewegung des Schieberelements ist ein Stopelement vorgesehen, das an dem ersten Griffteil schwenkbar angeordnet ist. Zudem ist es federbelastet. Das Stopelement begrenzt bei einem Ausführungsbeispiel die Schwenkbewegung des Mitnehmerteils. Bei einem weiteren Ausführungsbeispiel ist an dem ersten Griffteil ein Stoppelement angebracht, das das Schiebeelement in seiner Bewegung begrenzt. Es ist durch eine Schwenkbewegung aus seiner Stopposition zu bringen. Um das ungewollte Verlieren des Schiebeelements zu verhindern, ist vorgesehen, das Mitnehmerteil mit einem Sperrelement zu versehen.

### Nachteile des Standes der Technik

Die Wiederverwendbarkeit chirurgischer Instrumente mit Hilfe von geeigneten Sterilisationsverfahren ist bedeutend. Wiederverwendbarkelt Ist gleichzusetzen mit Wirtschaftlichkeit. Die durch Operation kontaminierten Instrumente sind nach ihrer Verwendung zu sterilisieren, um Gefährdung des menschlichen Lebens durch mikrobiologische oder nicht mikrobiologische Rückstände zu vermeiden. Es sind jedoch medizinisch chirurgische Instrumente aufgrund ihrer mechanischen Ausführung oder Funktion bekannt, die trotz eines anerkannten Sterilisationsverfahrens ungenügende Ergebnisse liefern. Dies sind insbesondere solche Geräte, die mechanisch zwar einwandfrei funktionieren, sich jedoch nicht genügend auseinander bauen lassen, damit auch beispielsweise Führungsflächen frei von Bakterien oder ähnlichem sind.

Insbesondere Conchotome haben die Eigenschaft, dass das Schiebeelement sich nicht von dem Führungselement trennen lässt, da ansonsten die Funktion nicht mehr gewährleistet ist. Zwischen dem Schiebeelement und dem Führungselement lagern sich Mikroorganismen an, die dann dazu führen, dass der gewünschte Sterilisationsgrad des Instrumentes nicht erreicht wird.

Andererseits, wird das Schiebeelement entfernt, besteht die Gefahr, dass nicht unmittelbar das seibe Schiebeelement wieder auf die selbe Führungsfläche aufgesetzt wird, da in der Regel bei Operationen mehrere Conchotome verwendet und somit auch sterilisiert werden.

Ist das Schiebelement Im Arbeitsbereich bereits fixiert, so ist es um diesen schwenkbar. Damit ist es möglich, die Bereiche des Schiebeelements zu reinigen, ohne die entsprechende Zuordnung zum Conchotom zu verlieren. Um zu verhindern, dass sich das Schiebeelement ungewollt vom übrigen Instrument löst, sind Sicherungselemente vorgesehen. Diese haben jedoch den Nachteil, dass diese sehr komplex aufgebaut sind, aus sehr vielen Teilen bestehen und in der Regel mit beiden Händen zu bedienen sind.

### Aufgabe der Erfindung

Die Aufgabe der Erfindung ist es, ein chirurgisches Instrument, beispielsweise ein Conchotom, derart konstruktiv weiterzuentwickeln, dass zumindest ein Nachteil des Standes der Technik vermieden wird.

### Lösung der Aufgabe

Der Grundgedanke der Lösung der Aufgabe ist es, die problematischen Geometrien, wie beispielsweise Spalten, Gleitflächen und Gewinde, für den Sterilisationsprozess frei zu geben und dennoch die Funktionalität des Instrumentes nach dem Sterilisationsprozess zu gewährleisten.

Unter Funktionalität wird die Eigenschaft verstanden, dass die Bauteile, die beim Herstellungsprozess aufeinander abgestimmt worden sind, auch nach dem Auseinanderbauen und Sterilisieren wieder problemlos zusammengefügt werden können, damit die gewünschte Eigenschaft des Funktionselements, beispielsweise der Zange oder der Schneide, erhalten bleibt.

Die Lösung wird gemäss der kennzeichnenden Merkmale des Anspruchs 1 vorgeschlagen.

### Vorteile der Erfindung

Der wesentliche Vorteil der Erfindung ist es, dass die an sich durch den Stand der Technik bekannten problematischen funktionalen Flächen des chirurgischen Instruments nun durch eine konstruktive Änderung für den Sterilisationsprozess freigegeben werden, aber dennoch die Funktionalität gewährleistet bleibt, Zudem ist die Sicherheit gegeben, dass sich das Schiebeelement nicht ungewollt von dem Instrument löst.

Das bei dem chirurgischen Instrument verwendete Schiebeelement bleibt unverlierbar mit dem Grundelement des chirurgischen Instrumentes verbunden, in dem es unverlierbar mit dem Funktionselement des chirurgischen Elements verbunden bleibt. Zur Freigabe der Führungsflächen zwischen dem Schiebeelement und dem Führungselement wird vorteilhafterweise das Schiebeelement in einer bestimmten Stellung des weiteren Griffteils von dem Kopplungselement entfernt und weggeklappt. Aufgrund der Anlenkung an dem Funktionselement bleibt aber dennoch die Funktionalität erhalten, da das exakt auf das chirurgische Instrument angepasste Schiebeelement insbesondere im Hinblick auf seine Passgenauigkeit unverlierbar mit dem selben chirurgischen Instrument verbunden bleibt.

Damit ein ungewolltes Entfernen des Schiebeelements nicht möglich ist, ist auf der proximalen Seite an dem ersten Griffteil, das Bestandteil des Grundelements ist, ein Begrenzungsmittel, ausgebildet als Schieber, angeordnet. Der Schieber ist derart ausgebildet, dass dieser die Schiebebewegung des Schiebeelements in proximaler Richtung beschränkt. Dadurch wird erreicht, dass der Öffnungswinkel zwischen den Griffteilen ebenfalls beschränkt wird.

Die Öffnungswinkel der beiden Griffteile teilen sich im wesentlichen in zwei Bereiche auf, nämlich einem ersten Bereich, in dem das Instrument genutzt wird und in einen zweiten Bereich, der die Entnahme des Schiebeelements ermöglicht. Der Öffnungswinkel des zweiten Bereichs ist daher grösser als der Öffnungswinkel des ersten Bereichs.

Das Begrenzungsmittel ist ein Bauteil, das integraler Bestandteil des Instruments und somit unverlierbar mit diesem verbunden ist. Es ist auf der proximalen Stirnseite des Instruments angeordnet und kann sehr einfach mit einem Finger, vorzugsweise einem Daumen, bedient werden. Um dieses zu bedienen, ist es nicht notwendig, die übliche Griffhaltung zu verlassen. Zudem kann die vorzugsweise geriffelte Oberfläche zusätzlich dazu genutzt werden, um das Instrument genauer zu führen und Druck mit dem Daumen in distaler Richtung auszuüben.

Bei Gebrauch des Instruments ist dieses Begrenzungsmittel derart angeordnet, dass es in einer ersten Ausführungsform den Schiebeweg des Schiebelements in proximaler Richtung beschränkt, in dem das Begrenzungsmittel zur Anlage an das Schiebeelement gelangt. Bei einem anderen Ausführungsbeispiel wird der Öffnungswinkel des beweglichen Griffteils beschränkt (dadurch indirekt auch der Schiebeweg des Schiebelements). Vorzugsweise geschieht dies im Bereich des Kopplungselements des Griffteils, das mit dem Schiebeelement zusammenwirkt. Grund hierfür ist, dass die auf das Begrenzungsmittel wirkendenden Kräfte klein sind. Ein weiterer Vorteil besteht darin, dass für den Benutzer die Sperrmechanik nicht ersichtlich ist.

Das Kopplungselement, das an dem weiteren Griffteil angeordnet ist und ein Mitnehmerteil umfasst, das mit einem Verbindungsteil an dem Schiebeelement korrespondiert, ist derart ausgelegt, dass eine Entkopplung während des Gebrauchs des Instruments nicht möglich ist. Erst durch aktives Betätigen des Schiebers wird eine grössere Auslenkung des Griffteils möglich, bis zu dem Punkt, an dem Mitnehmerteil und Verbindungsteil voneinander getrennt werden können.

Dadurch ist es möglich, das Schiebeelement aus dem Mitnehmerteil herauszuheben und um den Anlenkpunkt an dem Funktionsteil zu verschwenken. Das Mitnehmerteil weist vorzugsweise eine Schlitzführung auf, in die ein Stift, der mit dem Schiebeelement gekoppelt ist, eingreift. Der Stift erstreckt sich quer, vorzugsweise senkrecht, zur Längserstreckung des Schiebeelements. Dadurch ist erst eine Entnahme aus der Schlitzführung möglich, wenn das Griffteil in einer für den Gebrauch unüblichen Stellung ist und dadurch die Schlitzführung senkrecht zur Längserstreckung des Schiebeelements ist. Die Führungsflächen, die zwischen dem Führungselement und Schiebeelement vorhanden sind, werden so freigegeben und können problemlos gereinigt werden.

Vorzugsweise ist der Schieber federbelastet. Dies bedeutet und bringt auch den Vorteil mit sich, dass ein ungewolltes Lösen des Schiebeelements an sich nicht möglich ist. Erst durch aktive Betätigung, dass heisst durch Verschieben des Schiebers entgegen einer Federkraft, kann das Schiebeelement weiter in proximaler Richtung verschoben werden, so dass dann eine Entnahme aus dem Mitnehmerteil möglich ist. Somit kann sich der Benutzer darauf verlassen, dass beim Betätigen des Instruments keine ungewollte Loslösung des Schiebeelements eintritt. Vielmehr ist vorgesehen, dass bei nicht korrekter Kopplung des Schiebeelements mit dem Mitnehmerteil die Kopplung zwangsläufig durch Betätigen des Instruments herbeiführt wird.

Eine weitere vorteilhafte Ausbildung sieht vor, dass der Schieber nicht unmittelbar an dem proximalen Ende des Schiebeelements angreift, sondern innerhalb des Schiebeelements seine Begrenzung ausübt. Dies bringt den Vorteil mit sich, dass für die Betätigung des Schiebeelements, insbesondere für den Daumen, eine zusätzliche Drückfläche bereitgestellt wird, die das Bearbeiten mit dem chirurgischen Instrument weiter verbessert.

Um ein ungewolltes Abheben des Schiebeelements von dem Führungselement beziehungsweise dessen Führungsfläche zu vermeiden, sind auf der zur Führungsfläche hinweisenden Seite des Schiebeelements Führungsmittel vorgesehen. Diese Führungsmittel sind derart ausgestaltet, dass sie im Querschnitt vorzugsweise T-förmig ausgebildet sind und in der Führungsfläche vorgesehenen Hinterschneidung eingreifen. Dadurch wird ein Abheben senkrecht zur Längserstreckung des Schiebeelements vermieden.

Ausschliesslich in einer bestimmten Stellung, nämlich in der das Schiebeelement über seine Begrenzung auf proximaler Seite hinaus bewegt wird, sind auch die mit den Führungsmitteln korrespondierende Ausnehmungen in der Führungsfläche derart ausgestaltet, dass keine Hinterschneidungen mehr vorgesehen sind. Dadurch ist eine Entnahme des Schiebeelements von der Führungsfläche möglich. Damit liegt eine doppelte Sicherung vor, nämlich die Kopplung mit dem Mitnehmerteil und die schwalbenschwanzartige Führung des Schieberelements im Führungsmittel.

Weitere vorteilhafte Ausgestaltungen gehen aus der nachfolgenden Beschreibung, den Zeichnungen sowie den Ansprüchen hervor.

### Zeichnungen

Es zeigen:
- Figur 1: eine Seitenansicht auf ein erfindungsgemässes chirurgisches Instrument, nämlich ein Chonchotom im geschlossenen Zustand;
- Figur 2: eine Seitenansicht auf ein erfindungsgemässes chirurgisches Instrument, nämlich ein Conchotom mit geöffnetem Funktionselement;
- Figur 3: eine perspektivische Ansicht auf das in Figur 1 und 2 dargestellte chirurgisches Instrument, jedoch mit bereits abgenommenem Schiebeelement;
- Figur 4: einen Schnitt durch ein Teil des chirurgischen Instruments gemäss Figur 3;
- Figur 5: ein Teilausschnitt eines Längsschnittes durch das chirurgische Instrument gemäss Figur 1.

### Beschreibung eines Ausführungsbeispiels

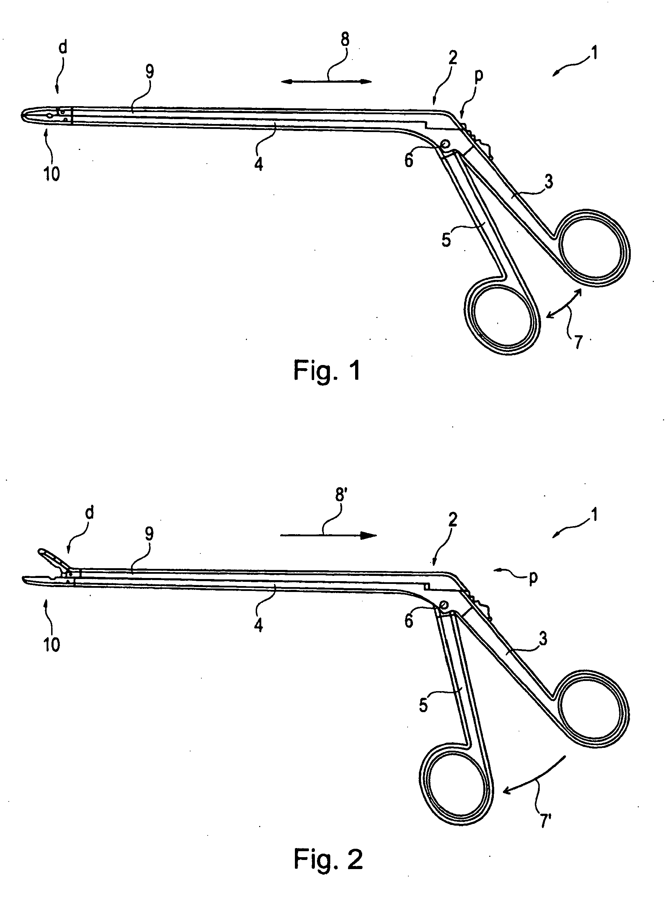

In Figur 1 ist eine Seitenansicht auf das erfindungsgemässe chirurgische Instrument 1 dargestellt. Das chirurgische Instrument 1 besteht im Wesentlichen aus einem Grundelement 2. Das Grundelement 2 besteht wiederum aus einem ersten Griffteil 3 und einem an dem Griffteil 3 verbundenen Führungselement 4. Das Führungselement 4 erstreckt sich von dem einen freien Ende des Griffteils 3 axial weg. Führungselement 4 und Griffteil 3 sind einstückig miteinander verbunden. Ferner ist ein weiteres Griffteil 5 vorgesehen, das an dem Grundelement 2 verschwenkbar ist. Um die Verschwenkbarkeit zu erreichen, ist ein Lagerelement 6 vorgesehen, das es ermöglicht, das weitere Griffteil 5 gegenüber dem ersten Griffteil 3 in und gegen Pfeilrichtung 7 zu verschwenken. Vorzugsweise ist das weitere Griffteil 5 unverlierbar (aber drehbeweglich) an dem Grundelement 2 angeordnet. Auf dem Führungselement 4 ist verschieblich in und gegen Pfeilrichtung 8 ein Schiebeelement 9 angeordnet. Dieses Schiebeelement 9 weist ein proximales Ende p und ein distales Ende d auf. An dem distalen Ende d des Schiebeelements 9 ist ein Funktionselement 10 vorgesehen, das sowohl mit dem Grundelement 2, insbesondere mit dem Führungselement 4, als auch mit dem Schiebeelement 9 gekoppelt ist. Bei dem hier dargestellten Ausführungsbeispiels stellt das chirurgische Instrument 1 ein Chonchotom dar, so dass das distale Ende d des chirurgischen Instruments 1 als Werkzeugmittel eine Schneideinrichtung vorsieht.

In Figur 2 ist das weitere Griffteil 5 in Pfeilrichtung 7' gegenüber dem ersten Griffteil 3 verschwenkt. Dadurch wird das Schiebeelement 9 in Pfeilrichtung 8' bewegt und das Funktionselement 10 öffnet sich. Dadurch ist ersichtlich, dass das weitere Griffteil 5 mit dem Schiebeelement 9 gekoppelt ist.

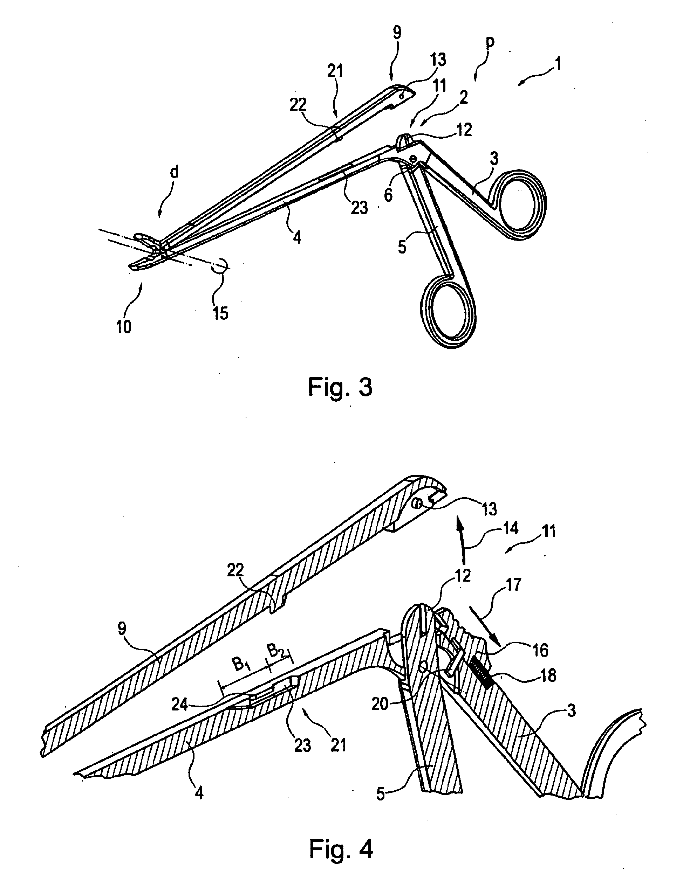

In Figur 3 ist gezeigt, wie das Schiebeelement 9 von dem Grundelement 2 entfernbar ist. Hierzu befindet sich das weitere Griffteil 5 in einer gesonderten Stellung, in der das Schiebeelement 9 aus dem Kopplungselement 11 und aus einem mit dem Mitnehmerteil 12 korrespondierenden Verbindungsteil 13 herausnehmbar ist.

In Figur 4 ist eine vergrösserte Darstellung, insbesondere des Kopplungselements 11, gezeigt. Das Mitnehmerteil 12 ist einstückig mit dem weiteren Griffteil 5 verbunden und weist an seinem freien Ende eine U-förmige Ausnehmung auf. In der in Figur 4 gezeichneten Stellung ist die U-förmige Ausnehmung nahezu senkrecht zur Längserstreckung des Führungselements 4 beziehungsweise des auf dem Führungselements 4 gelagerten Schiebeelements 9. Das Schiebeelement 9 ist bereits aus der U-förmigen Ausnehmung des Kopplungselements 11, insbesondere des Mitnehmerteils 12, in Pfeilrichtung 14 entnommen, wobei das distale Ende d des Schiebeelements 9 weiterhin mit dem Funktionselement 4 gekoppelt ist und um dieses, so wie es in Figur 3 gezeigt ist, zumindest eingeschränkt in oder gegen Pfeilrichtung 15 drehbeweglich zu verschwenken. Auf diese Weise ist es möglich, die Führungsfläche zwischen Schiebeelement 9 und Führungselement 4 zu reinigen, ohne dass das Schiebeelement 9, welches auf das Grundelement 2 angepasst ist, vollständig zu entfernen.

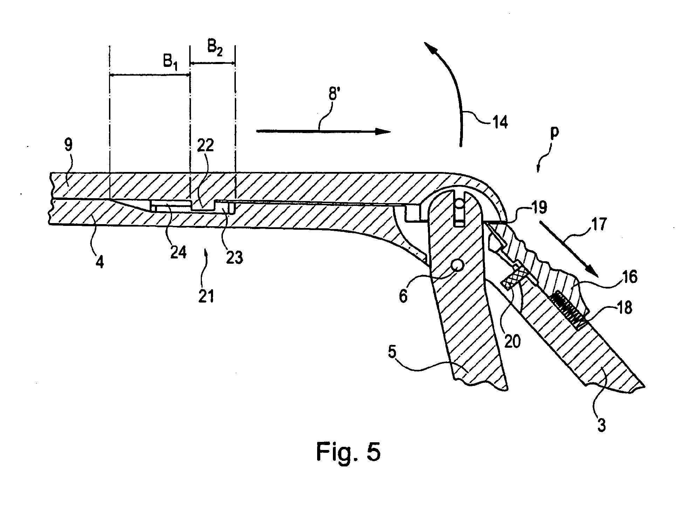

Um überhaupt das Schiebeelement 9, so wie es in Figuren 3 und 4 dargestellt ist, von dem Führungselement 4 in Pfeilrichtung 14 anzuheben, ist, wie bereits auch ausgeführt, das weitere Griffteil 5 in eine definierte Stellung zu bringen, die nahezu senkrecht zur Längserstreckung des Führungselements 4 ist (Figur 5). Diese Stellung kann jedoch nicht ohne weiteres erreicht werden, da verhindert werden soll, dass bei Anwendung des chirurgischen Instruments 1 das Schiebeelement 9 sich nicht ungewollt von dem Führungselement 4 lösen solite. Um dies zu erreichen ist ein Begrenzungsmittel 16 vorgesehen, das die Bewegung des Schiebeelements 9 in Pfeilrichtung 8' begrenzt. Um die Bewegung in Pfeilrichtung 8' freizugeben, ist es notwendig, dass das Begrenzungsmittel 16 in Pfeilrichtung 17 gegen eine Kraft einer Feder 18 verschoben wird, bis das freie Ende 19 des Begrenzungsmittels 16 das proximale Ende p des Schiebeelements 9 freigibt.

Übernimmt das Begrenzungsmittel 16 die Funktion, dass das Schiebeelement 9 nicht weiter als die in Figur 5 dargestellte Position verschoben wird, so ist das freie Ende 19 des Begrenzungsmittels 16 im Bereich des Schiebeelements 9 angeordnet und begrenzt zum einen die Schwenkbewegung des weiteren Griffteils 5 und/oder die des Schiebeelements 9.

Alternative Ausführungen zur Begrenzung der Bewegung des Schiebeelements 9 in Pfeilrichtung B' sind vielfältig. So ist auch beispielsweise denkbar, dass nicht das Schiebeelement 9, sondern das weitere Griffteil 5 in seiner Auslenkung beschränkt wird. Eine weitere Alternative sieht vor, dass ein zusätzliches Element, das an dem Führungselement 4 des Grundelements 2 angeordnet ist, die Bewegung einschränkt und je nach Bedarf durch Betätigung eines zusätzlichen Schiebers oder Schwenkmechanismus, diese dann zum weiteren Verschieben und dann zur Entnahme des Schiebeelements 9 freigibt.

Das in den Figuren beschriebene Begrenzungsmittel 16 ist zusätzlich mit seiner Bewegungseinrichtung durch ein Bolzenelement 20 eingeschränkt.

Um zu verhindern, dass das Schiebeelement 9 sich ungewollt von dem Führungselement 4 löst, sind die in den Figuren 3 bis 5 dargestellte Führungsmittel 21 vorgesehen. Ein solches Führungsmittel 21 besteht aus einem auf der zum Führungselement 4 hinweisenden Seite an dem Schiebeelement 9 angeordnetes erstes Führungsteil 22, das im Querschnitt vorzugsweise T-förmig ausgebildet ist. Dieses erste Führungsteil 22 korrespondiert mit einem weiteren Führungsteil 23, das auf der Seite des Führungselements 4, hinweisend zum Schiebeelement 9, angeordnet ist. Das weitere Führungsteil 23 ist eine Ausnehmung, die in einem definierten Bereich B1 eine Hinterschneidung 24 aufweist, die mit der T-förmigen Ausgestaltung des ersten Führungsteils 22 zusammenwirkt. Ferner ist ein weiterer Bereich B2 vorgesehen, der keine Hinterschneidung 24 aufweist und der so bemessen ist, dass das erste Führungsteil 22 vollständig aus dem weiteren Führungsteil 23 entnehmbar ist. Dies entspricht auch der Stellung, in der das Verbindungsteil 13 des Schiebeelements 9 aus der U-förmigen Ausgestaltung des Mitnehmerteils 12 entnehmbar ist.

Zum Zusammenfügen des Schiebeelements 9 mit dem Grundelement 2 ist es ausschliesslich notwendig, darauf zu achten, dass das zweite Griffteil 5 beziehungsweise dessen U-förmiges Mitnehmerteil 12 nahezu senkrecht zur Längserstreckung des Führungselements 4 ausgerichtet ist (wie in Figur 5 dargestellt), so dass das Verbindungsteil 13 des Schiebeelements 9 in die U-förmige Ausnehmung gedrückt werden kann. Gleichzeitig und vollkommen seibstständig wird das erste Führungsteil 22 in den Bereich B2 des zweiten Führungsteils 23 eingeführt und bereitet so die unverlierbare Verbindung zwischen dem Schiebeelement 9 und dem Führungselement 4 vor. Durch Bewegung des zweiten Griffteils 5 gegenüber dem ersten Griffteil 3 wird die Hin- und Herbewegung des Schiebeelements 9 erreicht und gleichzeitig tritt das Begrenzungsmittel 16 selbsttätig in Funktion.

Ist das chirurgische Instrument 1 in Gebrauch gewesen, so ist es ausschliesslich notwendig, das Begrenzungsmittel 16 in Pfeilrichtung 17 zu betätigen. Das zweite Griffteil 5 gegenüber dem ersten Griffteil 3 weit zu öffnen, in Pfeilrichtung 7' (Figur 2) und das Schiebeelement 9 in Pfeilrichtung 14 (Figur 5) mit dem Daumen zu drücken. Damit kann die in Figur 3 dargesteilte Position erreicht werden, so dass die Führungsflächen, die sich zwischen dem Schiebeelement 9 und dem Führungselement 4 bilden, ebenfalls durch den Sterilisationsprozess entsprechend gereinigt werden können.

Das Begrenzungsmittel 16 ist derart ausgelegt, dass es auf der Unterseite des Schiebeelements 9 eingreift. Die äussere Begrenzung dient dann als Anschlag. Ein weiteres Beispiel sieht vor, dass das Begrenzungsmittel 16 als Anschlagelement auf der proximalen Seite p des Schiebeelements 9 eingreift. Grundsätzlich dient das Begrenzungsmittel 16, unabhängig an welchem Bereich es angeordnet ist, dazu, den Verschiebeweg des Schiebeelements 9 zu begrenzen, derart, dass das Mitnehmerteil 12 nicht in die senkrechte Position zur Entnahme des Verbindungsteils 13 und damit des Schiebeelements 9 gelangt.

### BEZUGSZEICHENLISTE

- 1: chirurgisches Instrument
- 2: Grundelement
- 3: Griffteil
- 4: Führungselement
- 5: weiteres Griffteil
- 6: Lagerelement
- 7: Pfeilrichtung
- 7': Pfeilrichtung
- 8: Pfeilrichtung
- 8': Pfeilrichtung
- 9: Schiebeelement
- 10: Funktionselement
- 11: Kopplungselement
- 12: Mitnehmerteil
- 13: Verbindungsteil
- 14: Pfeilrichtung
- 15: Pfeilrichtung
- 16: Begrenzungsmittel
- 17: Pfeilrichtung
- 18: Feder
- 19: freies Ende
- 20: Bolzenelement
- 21: Führungsmittel
- 22: erstes Führungsteil
- 23: zweites Führungsteil
- 24: Hinterschneidung
- p: proximales Ende
- d: distales Ende
- B1: Bereich
- B2: Bereich

## Patentansprüche

1. Chirurgisches Instrument (1), bestehend aus
- einem Grundelement (2), das aus einem ersten Griffteil (3) sowie aus einem mit dem Griffteil verbundenen Führungselement (4) besteht, wobei das Führungselement sich von dem Griffelement aus axial erstreckt sowie
- einem weiteren Griffteil (5), das an dem Grundelement schwenkbar angeordnet ist,
- einem auf dem Führungselement in dessen axialer Erstreckung hin- und hergleitenden Schiebeelement (9) mit einem proximalen und einem distalen Ende, wobei das Schiebeelement an einem proximalen Ende mit einem weiteren Griffteil über ein Kopplungselement (11) verbunden ist,
- das Kopplungselement ein Mitnehmerteil (12) umfasst, das ein mit dem Mitnehmerteil korrespondierendes Verbindungsteil (13), das an dem Schiebeelement angeordnet ist, entsprechend der Schwenkbewegung des weiteren Griffteils hin- und herbewegt,
- das Schiebeelement (9) unverlierbar an dem distalen Ende (d) des Grundelementes (2) angeordnet ist,
- ein Begrenzungsmittel (16) vorgesehen ist, das am proximalen Ende (p) des Instruments, nämlich an dem ersten Griffteil (3) angeordnet ist und
- das Begrenzungsmittel (16) schieberartig ausgebildet ist,
**dadurch gekennzeichnet, dass** das Begrenzungsmittel (16) derart verschieblich angeordnet ist, dass es die Schwenkbewegung (7, 7') des weiteren Griffteils (5) beschränkt, indem das Begrenzungsmittel (16) auf der Unterseite des Schiebeelements (9) eihgreift.

2. Chirurgisches Instrument nach Anspruch 1, **dadurch gekennzeichnet, dass** das Begrenzungsmittel (16) mit einer Feder (18) federbelastet ist, derart, dass es in seine Sperrstellung seibsttätig verfährt.

3. Chirurgisches Instrument nach Anspruch 2, **dadurch gekennzeichnet, dass** die Federkraft der Feder (18) auf das Begrenzungsmittel (16) in Richtung des Schlebestements (9) wirkt.

4. Chirurgisches Instrument nach mindestens einem der vorhergahsnden Ansprüche, **dadurch gekennzeichnet, dass** zwischen dem proximalen Ende (p) und dem distalen Ende (d) das Schiebeelements (9) ein Führungsmittel (21) vorgesahen ist, das mit dem Führungselement (4) korrespondiert.

5. Chirurgisches Instrument nach Anspruch 4, **dadurch gekennzeichnet, dass** zwischen dem Schiebeelement (9) und dem Führungselement (4) eine Führungsfläche vorgssehen ist, wobei sich von dem Schiebeelement (9) weg mindestens ein T-förmiges erstes Führungstell (22) erstreckt, das mit einem weiteren in der Führungsfläche des Führungselements (4) vorgesehenen zweiten Führungsteil (23) zusammenwirkt.

6. Chirurgisches Instrtrument nach Anspruch 5, dadurch gekennzeicht, dass das zweite Führungsteil (23) derart ausgestaltet ist, dass über einen definierten Bereich (B1) das T-förmige erste Führungsteil (22) diesen Bereich (B1) hinterschneidet und ab einer definierten Stellung des weiteren Griffteils (5) zum ersten Griffteil (3) ein weiterer Bereich (B2) vorgesehen ist, bei dem das erste Führungsteil (22) entfernbar ist.

## Claims

1. Surgical instrument (1) composed of
- a main element (2) which is composed of a first grip part (3) and of a guide element (4) connected to the grip part, wherein the guide element extends axially proceeding from the grip element, and
- a further grip part (5) which is arranged pivotably on the main element,
- a slide element (9) which can slide back and forth on the guide element in the direction of the axial extent thereof and which has a proximal end and a distal end, wherein the slide element is connected at a proximal end to a further grip part via a coupling element (11),
- the coupling element comprises a driver part (12) by means of which a connecting part (13), which corresponds to the driver part and which is arranged on the slide element, is moved back and forth correspondingly to the pivoting movement of the further grip part,
- the slide element (9) is arranged captively on the distal end (d) of the main element (2),
- a limitation means (16) is provided which is arranged on the proximal end (p) of the instrument, specifically on the first grip part (3), and
- the limitation means (16) is formed in the manner of a slide,
**characterized in that**
the limitation means (16) is arranged in a displaceable manner so as to restrict the pivoting movement (7, 7') of the further grip part (5), by virtue of the fact that the limitation means (16) engages on the underside of the slide element (9).

2. Surgical instrument according to Claim 1, **characterized in that** the limitation means (16) is spring-loaded by means of a spring (18) such that said limitation means travels automatically into its blocking position.

3. Surgical instrument according to Claim 2, **characterized in that** the spring force of the spring (18) acts on the limitation means (16) in the direction of the slide element (9).

4. Surgical instrument according to at least one of the preceding claims, **characterized in that** a guide means (21) which corresponds with the guide element (4) is provided between the proximal end (p) and the distal end (d) of the slide element (9).

5. Surgical instrument according to Claim 4, **characterized in that** a guide surface is provided between the slide element (9) and the guide element (4), wherein a T-shaped first guide part (22) extends away from the slide element (9), which first guide part interacts with a further, second guide part (23) provided in the guide surface of the guide element (4).

6. Surgical instrument according to Claim 5, **characterized in that** the second guide part (23) is designed such that, over a defined region (B1), the T-shaped first guide part (22) engages behind said region (B1)and, beyond a defined position of the further grip part (5) relative to the first grip part (3), a further region (B2) is provided in which the first guide part (22) is removable.

## Revendications

1. Instrument chirurgical (1), constitué :
- d'un élément de base (2) qui se compose d'une première partie de préhension (3) ainsi que d'un élément de guidage (4) connecté à la partie de préhension, l'élément de guidage s'étendant axialement depuis l'élément de préhension et
- d'une autre partie de préhension (5) qui est disposée de manière à pouvoir pivoter sur l'élément de base,
- d'un élément coulissant (9) pouvant glisser d'avant en arrière dans son étendue axiale sur l'élément de guidage, avec une extrémité proximale et une extrémité distale, l'élément coulissant étant connecté au niveau d'une extrémité proximale à une autre partie de préhension par le biais d'un élément d'accouplement (11),
- l'élément d'accouplement comprenant une partie d'entraînement (12) qui déplace, en fonction du mouvement de pivotement de l'autre partie de préhension, d'avant en arrière une partie de connexion (13) correspondant à la partie d'entraînement, laquelle est disposée sur l'élément coulissant,
- l'élément coulissant (9) étant disposé de manière imperdable sur l'extrémité distale (d) de l'élément de base (2),
- un moyen de limitation (16) étant prévu, lequel est disposé à l'extrémité proximale (p) de l'instrument, à savoir au niveau de la première partie de préhension (3) et
- le moyen de limitation (16) étant réalisé sous forme de coulisseau,
**caractérisé en ce que**
le moyen de limitation (16) est disposé de manière coulissante de telle sorte qu'il limite le mouvement de pivotement (7, 7') de l'autre partie de préhension (5), par le fait que le moyen de limitation (16) s'engage sur le côté inférieur de l'élément coulissant (9).

2. Instrument chirurgical selon la revendication 1, **caractérisé en ce que** le moyen de limitation (16) est sollicité par ressort avec un ressort (18), de telle sorte qu'il se déplace automatiquement dans sa position de verrouillage.

3. Instrument chirurgical selon la revendication 2, **caractérisé en ce que** la force de ressort du ressort (18) agit sur le moyen de limitation (16) dans la direction de l'élément coulissant (9).

4. Instrument chirurgical selon au moins l'une quelconque des revendications précédentes, **caractérisé en ce qu'**entre l'extrémité proximale (p) et l'extrémité distale (d) de l'élément coulissant (9) est prévu un moyen de guidage (21) qui correspond à l'élément de guidage (4).

5. Instrument chirurgical selon la revendication 4, **caractérisé en ce qu**'entre l'élément coulissant (9) et l'élément de guidage (4) est prévue une surface de guidage, au moins une première partie de guidage en forme de T (22) s'étendant depuis l'élément coulissant (9), laquelle partie de guidage coopère avec une deuxième partie de guidage supplémentaire (23) prévue dans la surface de guidage de l'élément de guidage (4).

6. Instrument chirurgical selon la revendication 5, **caractérisé en ce que** la deuxième partie de guidage (23) est configurée de telle sorte que sur une région définie (B1), la première partie de guidage en forme de T (22) forme une contre-dépouille avec cette région (B1)et à partir d'une position définie de l'autre partie de préhension (5) par rapport à la première partie de préhension (3), une région supplémentaire (B2) soit prévue, dans laquelle la première partie de guidage (22) peut être enlevée.
